Europäisches Patentamt

European Patent Office

Office européen des brevets

(11) Veröffentlichungsnummer: **0 222 986**
**A1**

(12) # EUROPÄISCHE PATENTANMELDUNG

(21) Anmeldenummer: 86111564.0

(22) Anmeldetag: 21.08.86

(51) Int. Cl.4: **G01N 29/02** , **G10K 11/00** , **A61M 5/14**

Die Bezeichnung der Erfindung wurde geändert (Richtlinien für die Prüfung im EPA, A-III, 7.3).

(30) Priorität: 28.08.85 DE 3530747

(43) Veröffentlichungstag der Anmeldung:
27.05.87 Patentblatt 87/22

(84) Benannte Vertragsstaaten:
AT BE CH DE FR GB IT LI LU NL SE

(71) Anmelder: **Stöckert Instrumente GmbH**
**Osterwaldstrasse 10**
**D-8000 München 40(DE)**
Anmelder: **Scheller, Thomas, Dipl.-Ing.**
**Bunzlauer Platz 1**
**D-8000 München 50(DE)**

(72) Erfinder: **Scheller, Thomas, Dipl.-Ing.**
**Bunzlauer Platz 1**
**D-8000 München 50(DE)**
Erfinder: **Heinze, Werner, Ing. grad.**
**Höhenstrasse 6**
**D-8911 Windach(DE)**
Erfinder: **Schreyer, Johann, Dipl.-Ing.**
**Pfarrer-Grimm-Strasse 10b**
**D-8000 München 50(DE)**
Erfinder: **Wysocki, Roman, Dipl.-Ing.**
**Wachterstrasse 15**
**D-8170 Bad Tölz(DE)**

(74) Vertreter: **Hansen, Bernd, Dr.rer.nat. et al**
**Hoffmann, Eitle & Partner Patentanwälte**
**Arabellastrasse 4**
**D-8000 München 81(DE)**

(54) **Ultraschallsensor zum Detektieren von Luftblasen in fliessenden Flüssigkeiten.**

(57) Es wird ein Ultraschallsensor zum nichtinvasiven Detektieren von Luftblasen in durch einen Schlauch - (80) fließenden Flüssigkeiten vorgeschlagen, der im wesentlichen aus zwei zusammenfügbaren Gehäusehälften (10) besteht. Zur akustischen Ankopplung des Schlauches (80) an die beiden einander gegenüberliegenden Ultraschallwandler (20) weist jede Gehäusehälfte (10) eine Kammer (40) auf, die von einer flexiblen Membran (30) abgeschlossen wird und mit einer gut Schall übertragenden Flüssigkeit (50) gefüllt ist. Die durch den zwischen die beiden Gehäusehälften (10) eingelegten Schlauch (80) verdrängte Flüssigkeit (50) preßt die Membran - (30) beidseitig in Form eines Wulstes (33) hoch. Hierdurch wird ein ausgezeichneter Kontakt zwischen der mit der Flüssigkeit (50) gefüllten Kammer (40) und dem Schlauch (80) unabhängig von dessen Durchmesser hergestellt. Die Membran (30) kann in die Seitenwände (12, 13) eingelassen und entlang dieser tonnenförmig gewölbt sein.

# Fig. 2

## Ultraschallsensor

Die Erfindung betrifft einen Ultraschallsensor zum nichtinvasiven Detektieren von Luftblasen in durch einen Schlauch fließenden Flüssigkeiten, mit zwei zusammenfügbaren Gehäusehälften, in die der Schlauch eingelegt wird, mit zwei jeweils in eine der Gehäusehälften eingebauten, einander gegenüberliegenden Ultraschallwandlern, von denen der eine als Sender und der andere als Empfänger dient, sowie mit jeweils vor den Ultraschallwandlern angeordneten Vorlaufstrecken zur akustischen Ankopplung des zwischengelegten, flüssigkeitsführenden Schlauches.

Derartige Ultraschallsensoren werden überall dort verwendet, wo eine in einem Schlauch oder Rohr fließende Flüssigkeit auf das Vorhandensein von Luftblasen kontrolliert werden muß, ohne daß in den Flüssigkeitskreislauf eingegriffen werden darf. Beispielsweise muß ein extrakorporaler Blutkreislauf fortwährend und mit höchster Zuverlässigkeit überwacht werden, damit keine für den Patienten gefährliche Luftblasen ins Körperblut gelangen. Zum Detektieren von Gasblasen wird der flüssigkeitsführende Schlauch zwischen die beiden Ultraschallwandler gebracht. Ist der Schlauch vollständig mit Flüssigkeit ausgefüllt, so werden die von dem einen Ultraschallwandler ausgehenden Schallwellen ungehindert zu dem gegenüberliegenden, als Empfänger dienenden Wandler hin übertragen. Passieren jedoch Gasblasen das Schallfeld, so werden die Schallwellen hierdurch gedämpft, was zu deutlichen Amplitudenänderungen des Empfangssignals führt.

Nach diesem Prinzip arbeitende Ultraschallsensoren sind bereits in verschiedenen Ausführungen bekannt. In der europäischen Veröffentlichungsschrift 0053 453 ist beispielsweise ein Ultraschallsensor beschrieben, bei dem die beiden einander gegenüberliegenden Ultraschallwandler gemeinsam in ein einstückiges Gehäuse eingebaut sind. Vor den Ultraschallwandlern sind elastische Vorlaufstrecken angeordnet, zwischen die der flüssigkeitsführende Schlauch eingeklemmt werden kann. Es sind auch Ausführungen mit zwei, von beiden Seiten an den Schlauch anlegbaren Gehäusehälften bekannt, bei denen zur akustischen Ankopplung ebenfalls Vorlaufstrecken aus elastischem Material vorgesehen sind (z.B. US-Patent 3 663 842). Diese bekannten Ultraschallsensoren haben den Nachteil, daß ihre Verwendung auf einen einzigen bestimmten Schlauchdurchmesser beschränkt ist oder deren mehr oder weniger elastischen Klemmkörper den flüssigkeitsführenden Schlauch an der Meßstelle zusammendrücken. Eine lokale Querschnittsveränderung führt sofort zu einem starken Anstieg der Fließgeschwindigkeit und zu Turbulenzen im Flüssigkeitsstrom. Als Folge treten Meßfehler sowie unerwünschte Veränderungen in der zu überwachenden Flüssigkeit auf. Beispielsweise besteht die Gefahr, daß Blutkörperchen zerstört werden. Wegen des mangelnden mechanischen Kontakts zwischen Schlauch und Vorlaufstrecke ist die akustische Kopplung zwischen den Ultraschallwandlern und dem flüssigkeitsführenden Schlauch überdies unbefriedigend. Durch die Verwendung von sogenanntem Ultraschallkoppel-Gel kann dies nur unvollkommen ausgeglichen werden.

Vorliegender Erfindung liegt daher die Aufgabe zugrunde, einen Ultraschallsensor der erwähnten Art so auszugestalten, daß er unter Vermeidung der geschilderten Nachteile an Schläuche unterschiedlichen Durchmessers einfach angelegt werden kann, ohne daß dabei der Schlauch wesentlich verformt wird, und insbesondere eine gute akustische Ankopplung gewährleistet ist.

Bei der Lösung dieser Aufgabe wird ausgegangen von einem Ultraschallsensor zum nichtinvasiven Detektieren von Luftblasen in durch einen Schlauch fließenden Flüssigkeiten mit zwei zusammenfügbaren Gehäusehälften, in die der Schlauch eingelegt wird, mit zwei jeweils in eine der Gehäusehälften eingebauten, einander gegenüberliegenden Ultraschallwandlern, von denen der eine als Sender und der andere als Empfänger dient, sowie mit jeweils vor den Ultraschallwandlern angeordneten Vorlaufstrecken zur akustischen Ankopplung des zwischengelegten, flüssigkeitsführenden Schlauches, und gelöst wird sie dadurch, daß die Vorlaufstrecken aus einer mit einer gut Schall übertragenden Flüssigkeit gefüllten und von einer flexiblen Membran abgeschlossenen Kammer gebildet werden. Wird der erfindungsgemäß ausgestaltete Ultraschallsensor an einen Schlauch angelegt, so preßt die verdrängte Flüssigkeit in den Kammern die flexible Membran in Form eines Wulstes zu beiden Seiten des Schlauches hoch.

Hierdurch wird ein großflächiger, sicherer Kontakt zum Schlauch hergestellt. Über die in der Kammer eingeschlossene, den Schall gut leitende Flüssigkeit wird eine optimale akustische Ankopplung des betreffenden Ultraschallwandlers erzielt. Auf die Verwendung von Ultraschallkoppel-Gel kann verzichtet werden. Da sich die flexible Membran wie ein weiches Kissen um den Schlauch herumlegt, wird dieser nicht oder allenfalls unwesentlich verformt, so daß unerwünschte Turbulenzen im Flüssigkeitsstrom vermieden werden. Die gestellte Aufgabe ist somit gelöst.

Bei einer bevorzugten Ausführung des erfindungsgemäßen Ultraschallwandlers wird die mit der Flüssigkeit gefüllte Kammer von den Seitenwänden der im wesentlichen U-förmigen Querschnitt aufweisenden Gehäusehälften, der zwischen diesen Seitenwänden gespannten Membran sowie von dem am Gehäuseboden angeordneten Ultraschallwandler umgrenzt. Bei dem so ausgestalteten Ultraschallsensor sind die die Vorlaufstrecken darstellenden, mit der gut Schall leitenden Flüssigkeit gefüllten Kammern in die Gehäusehälften integriert. Der U-förmige Querschnitt der Gehäusehälften ergibt zwangsläufig einen wannenförmigen Hohlraum, der lediglich noch von der zwischen den Seitenwänden gespannten Membran abgeschlossen werden muß. Der vorgeschlagene Ultraschallsensor ist somit einfach und kostengünstig herzustellen. Außerdem wird ein zwischen die beiden Gehäusehälften eingebrachter, auf den flexiblen Membranen aufliegender Schlauch von den hochgezogenen, die U-Schenkel darstellenden Seitenflächen auch seitlich und somit allseitig geführt.

Bei einer bevorzugten Ausführung ist die Membran entlang der Seitenwände tonnenförmig, insbesondere kreisbogenförmig gewölbt. Dies führt zu einer gleichmäßigen Verteilung des Anpreßdrucks auf den anliegenden Schlauch und gestattet die Verwendung bei unterschiedlichen Schlauchdurchmessern. Die Wölbung der Membran kann im Bereich um den Scheitelpunkt abgeflacht sein, um die ebene Kontaktfläche mit dem eingelegten Schlauch zu vergrößern. Dies kann beispielsweise durch Reduzieren des Flüssigkeitsdrucks innerhalb der Kammer erreicht werden.

Eine gute Abdichtung der flüssigkeitsgefüllten Kammer wird erzielt, wenn die Seitenränder der Membran in die Seitenwände der Gehäusehälfte eingelassen sind.

Bei einer besonders vorteilhaften Ausführung des erfindungsgemäßen Ultraschallsensors werden die Seitenwände der Gehäusehälften von einem einstückig angeformten Teil, das eine Ausnehmung aufweist, sowie einem entsprechend dieser Ausnehmung geformten, anmontierten Seitenteil gebildet und ist die Membran mit ihren Seitenrändern zwischen dem angeformten Teil und dem Seitenteil eingespannt. Die zweistückige Ausbildung der Seitenwände erlaubt eine besonders einfache und flüssigkeitsdichte Montage der flexiblen Membran. Die gewünschte kreisbogenförmige Wölbung erhält die Membran, wenn die anmontierten Seitenteile die Gestalt eines Kreisabschnitts aufweisen und die Seitenränder der Membran zwischen diese Seitenteile und die eine entsprechende kreisförmige Ausnehmung aufweisenden, feststehenden Teile der Seitenwände eingelegt sind.

Zur Aufnahme der beim Einlegen eines Schlauches verdrängten, von der Membran eingeschlossenen Flüssigkeit können rings um die Membran Nuten in den Gehäusehälften vorgesehen sein. Die auf diese Weise geschaffenen Freiräume erlauben ein seitliches Ausweichen des Flüssigkeitskissens und damit eine besonders gute Anpassung an unterschiedliche Schlauchdurchmesser. Zweckmäßig besitzen die Nuten in den Seitenwänden rechteckigen Querschnitt, während die Nuten im Gehäuseboden der Wölbung der Membran angepaßt sind und dreieckigen Querschnitt aufweisen.

Bei einer besonders vorteilhaften Ausführung des erfindungsgemäßen Ultraschallsensors sind bei beiden Gehäusehälften oberhalb der Membran besondere Führungsleisten vorgesehen, welche von den Seitenwänden nach innen weisen. Sind diese Führungsleisten bis an den eingelegten Schlauch seitlich heranreichend ausgeführt, so gewährleisten sie eine stets exakte Ausrichtung des Schlauches in bezug auf die in die obere bzw. untere Gehäusehälften eingebauten Ultraschallwandler. Ein seitliches Ausweichen des Schlauches, beispielsweise auch infolge flexibler Verformung desselben, wird durch diese seitliche Fixierung wirksam verhindert. Der gesamte, flüssigkeitsdurchströmte Querschnitt des Schlauches befindet sich deshalb immer innerhalb des Ultraschallfeldes; selbst bei einem unachtsam eingelegten Schlauch oder einem Schlauch zu großen Durchmessers ist eine einwandfreie Blasendetektion gewährleistet. Da ein Schlauch mit etwas zu großem Durchmesser seitlich nicht mehr ausweichen und somit allenfalls nur noch die Querschnittsform eines aufrecht stehenden Ovals annehmen kann, verhindern die Führungsleisten überdies eine sonst mögliche Einschnürung der Schlauchwände, welche im ungünstigsten Fall sogar zu einem direkten Kontakt der beiden gegenüberliegenden Membranen und damit zu einem akustischen Kurzschluß führen könnte. Unabhängig von der genauen Einhaltung eines bestimmten Durchmessers des verwendeten Schlauches und dessen sorgfältigem Einlegen zwischen die beiden Gehäusehälften arbeitet ein mit den erwähnten Führungsleisten ausgerüsteter Ultraschallsensor somit stets zuverlässig und störungsfrei. Eine leichte Biegung der Führungsleisten über ihre Längsrichtung erleichtert der flexiblen Membran das Anlegen an die Schlauchaußenwand über einen möglichst langen Abschnitt.

Zur Erzielung einer hohen Nachgiebigkeit ist die Membran vorzugsweise aus einem gummielastischen Kunststoff gefertigt.

In zweckmäßiger Ausgestaltung der Erfindung sind die beiden Gehäusehälften mittels eines Gelenkes aufklappbar miteinander verbunden. Dies erleichtert das Einlegen eines Schlauches, in dem die auf Luftblasen hin zu überwachende Flüssigkeit fließt, in den Ultraschallsensor. Von Vorteil ist ein an den dem Gelenk gegenüberliegenden Seiten der Gehäusehälften angebrachter Verschluß, mit dem sich der Ultraschallsensor nach Einlegen des Schlauches verriegeln läßt.

Ausführungsbeispiele der Erfindung werden nachstehend anhand der beigefügten Zeichnungen erläutert. Es zeigen:

Fig. 1 einen erfindungsgemäßen Ultraschallsensor mit zwei durch ein Gelenk verbundenen Gehäusehälften von U-förmigem Querschnitt, in einem Vertikalschnitt;

Fig. 2 den Ultraschallsensor gemäß Fig. 1 mit einem zwischen die Gehäusehälften eingelegten Schlauch, ebenfalls in einem Vertikalschnitt;

Fig. 3 eine U-förmigen Querschnitt aufweisende Gehäusehälfte eines erfindungsgemäßen Ultraschallsensors mit halbkreisförmig gewölbter Membran, zusätzlichen Nuten und Führungsleisten, sowie ein abgeschnittenes Stück eines einzulegenden Schlauches, in einer perspektivischen Ansicht;

Fig. 4 die Gehäusehälfte gemäß Fig. 3, in einem Vertikalschnitt entlang der Linie A-A;

Fig. 5 die Gehäusehälfte gemäß Fig. 3 mit eingelegtem Schlauch, in einem Vertikalschnitt entlang der Linie B-B.

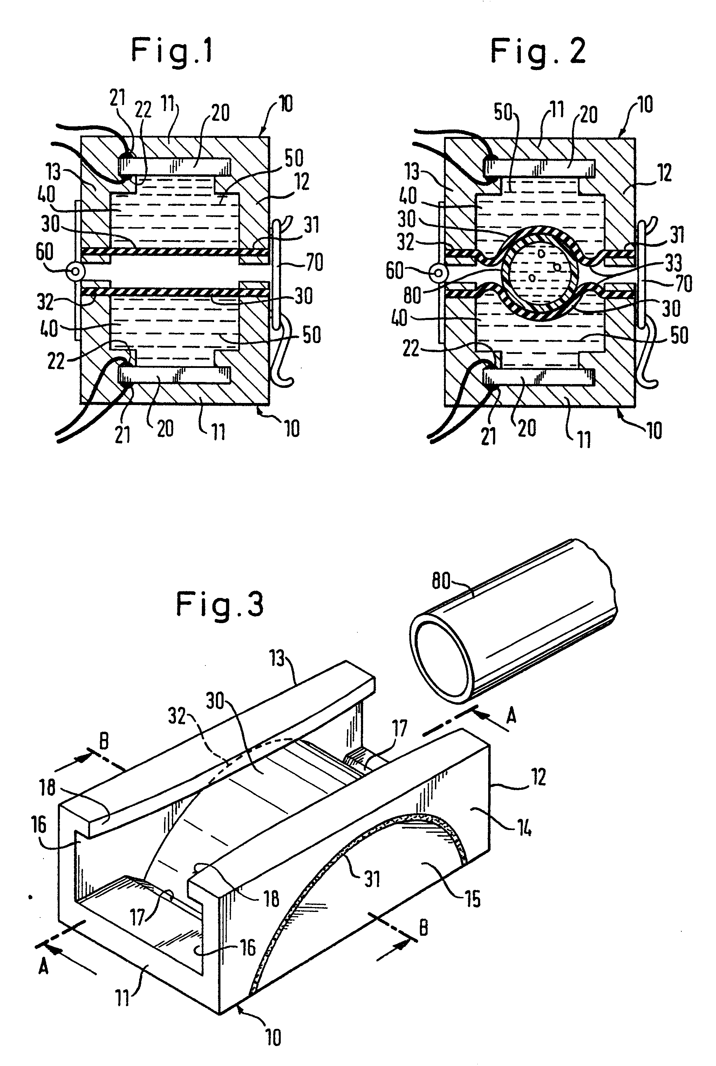

Der in Fig. 1 dargestellte Ultraschallsensor besteht aus zwei im wesentlichen gleichen Gehäusehälften 10. An deren Gehäuseböden 11 ist jeweils ein Ultraschallwandler 20 angeordnet. Diese bestehen aus Piezo-Keramik und sind jeweils an ihrer Ober-und Unterseite mit elektrischen Anschlußkontakten 21, 22 versehen. Zwischen den Seitenwänden 12 und 13 der Gehäusehälften 10 ist jeweils eine Membran 30 aus gummielastischem Kunststoff gespannt. Die Gehäusehälften 10 besitzen U-förmigen Querschnitt. Ihre Seitenwände 12, 13 bilden zusammen mit den Ultraschallwandlern 20 an den Gehäuseböden 11 jeweils eine Kammer 40, welche von der flexiblen Membran 30 abgeschlossen wird. Das Innere der Kammer 40 ist vollständig mit einer gut Schall übertragenden Flüssigkeit 50 ausgefüllt. Ein Gelenk 60 verbindet die beiden Gehäusehälften 10. Auf der gegenüberliegenden Seite ist ein Verschluß 70 angebracht.

Fig. 2 zeigt einen in den Ultraschallsensor eingelegten Schlauch 80, durch den die auf eventuell vorhandene Gasblasen zu überwachende Flüssigkeit fließt. Die durch den Schlauch 80 verdrängte Flüssigkeit 50 preßt die Membranen 30 beidseitig in Form von Wülsten 33 hoch. Die Membranen 30 liegen somit jeweils etwa auf dem halben Umfang des Schlauches 80 ohne Zwischenraum an. Die Kammern 40 stellen Vorlaufstrecken vor den Ultraschallwandlern 20 dar. Die von dem einen, als Sender dienenden Ultraschallwandler 20 ausgehenden Schallwellen werden durch die gut Schall leitende Flüssigkeit 50 weitgehend ungehindert auf den Schlauch 80 übertragen. Eventuell auftretende Gasblasen in der durch den Schlauch 80 fließenden Flüssigkeit setzen die Intensität der von dem gegenüberliegenden, als Empfänger dienenden Ultraschallwandler 20 empfangenen Schallwellen stark herab. Der großflächige Kontakt zwischen der von der Membran 30 eingeschlossenen Flüssigkeit 50 und dem Schlauch 80 gewährleistet eine hervorragende akustische Ankopplung der Ultraschallwandler 20.

Bei der in Fig. 3 dargestellten Gehäusehälfte 10 ist die Membran 30 entlang den Seitenwänden 12, 13 kreisbogenförmig gewölbt. Jede Seitenwand 12, 13 weist ein fest angeformtes Teil 14 mit einer kreisbogenförmigen Ausnehmung auf, in das ein entsprechend kreisabschnittsförmig ausgebildetes Seitenteil 15 eingesetzt ist.

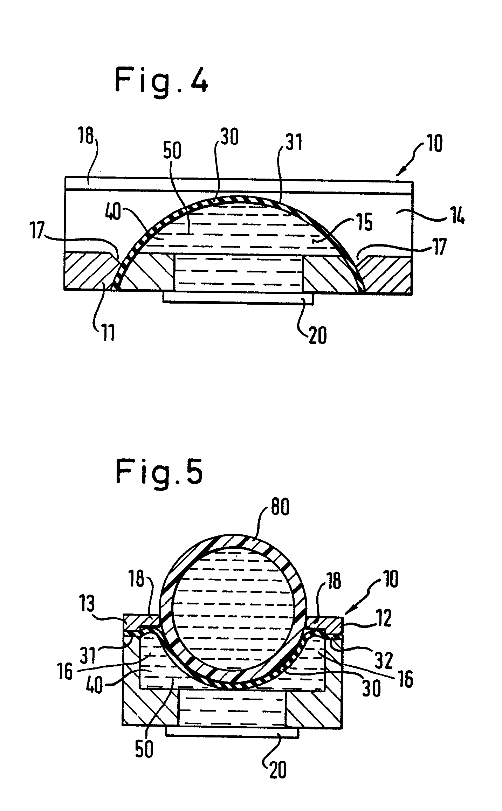

Wie insbesondere aus den Fig. 3, 4 und 5 ersichtlich ist, sind die Seitenränder 31, 32 der Membran 30 zwischen dem angeformten Teil 14 und dem Seitenteil 15 eingespannt. In die Seitenwände 12, 13 sind innen Nuten 16 von rechteckigem Querschnitt eingearbeitet. Am Gehäuseboden 11 sind Nuten 17 von dreieckigem Querschnitt vorgesehen. Die durch diese Nuten 16, 17 geschaffenen Freiräume dienen der Aufnahme der beim Einlegen des Schlauches 80 verdrängten, von der Membran 30 eingeschlossenen Flüssigkeit 50 (vergl. Fig. 5). Die Seitenwände 12, 13 dienen gleichzeitig der Längsführung des Schlauches 80. Es sind besondere Führungsleisten 18 vorgesehen, welche von den Seitenwänden 12, 13 oberhalb der Membran 30 nach innen vorstehen. Die Führungsleisten 18 reichen bis an den eingelegten Schlauch 80 seitlich heran, so daß dieser nicht nach rechts und links, sondern allenfalls nach oben bzw. unten ausweichen kann. Die Führungsleisten 18 sind in Längsrichtung leicht gebogen (vgl. Fig. 3).

**Ansprüche**

1. Ultraschallsensor zum nichtinvasiven Detektieren von Luftblasen in durch einen Schlauch fließenden Flüssigkeiten, mit zwei zusammenfügbaren Gehäusehälften, in die der Schlauch eingelegt wird, mit zwei jeweils in eine der Gehäusehälften eingebauten, einander gegenüberliegenden Ultraschallwandlern, von denen der eine als Sender und der andere als Empfänger dient, sowie mit jeweils vor den Ultraschallwandlern angeordneten Vorlaufstrecken zur akustischen Ankopplung des zwischengelegten, flüssigkeitsführenden Schlauches, dadurch **gekennzeichnet**, daß die Vorlaufstrecken jeweils aus einer mit einer gut Schall übertragenden Flüssigkeit (50) gefüllten und von einer flexiblen Membran (30) abgeschlossenen Kammer (40) gebildet werden.

2. Ultraschallsensor nach Anspruch l, dadurch gekennzeichnet, daß die mit der Flüssigkeit (50) gefüllte Kammer (40) von den Seitenwänden (l2, l3) der im wesentlichen U-förmigen Querschnitt aufweisenden Gehäusehälfte (l0), der zwischen diesen Seitenwänden (l2, l3) gespannten Membran - (30) sowie von dem am Gehäuseboden (ll) angeordneten Ultraschallwandler (20) umgrenzt wird.

3. Ultraschallsensor nach Anspruch 2, dadurch gekennzeichnet, daß die Membran (30) entlang der Seitenwände (l2, l3) tonnenförmig gewölbt ist.

4. Ultraschallsensor nach Anspruch 3, dadurch gekennzeichnet, daß die Wölbung der Membran - (30) kreisbogenförmig verläuft.

5. Ultraschallsensor nach Anspruch 3, dadurch gekennzeichnet, daß die Wölbung der Membran - (30) im bereich um den Scheitelpunkt abgeflacht ist.

6. Ultraschallsensor nach einem der Ansprüche 2 bis 5, dadurch gekennzeichnet, daß die Seitenränder (3l, 32) der Membran (30) in die Seitenwände (l2, l3) der Gehäusehälfte (l0) eingelassen sind.

7. Ultraschallsensor nach einem der Ansprüche 2 bis 6, dadurch gekennzeichnet, daß die Seitenwände (l2, l3) jeder Gehäusehälfte (l0) von einem einstückig angeformten Teil (l4), der eine Ausnehmung aufweist, sowie einem entsprechend dieser Ausnehmung geformten, anmontierten Seitenteil (l5) gebildet werden, und daß die Membran - (30) mit ihren Seitenrändern (3l, 32) zwischen dem angeformten Teil (l4) und dem Seitenteil (l5) eingespannt ist.

8. Ultraschallsensor nach Anspruch 4 sowie Anspruch 7, dadurch gekennzeichnet, daß die anmontierten Seitenteile (l5) die Gestalt eines Kreisabschnitts aufweisen.

9. Ultraschallsensor nach einem der Ansprüche l bis 8, dadurch gekennzeichnet, daß rings um die Membran (30) Nuten (l6, l7) in jeder Gehäusehälfte (l0) zur Aufnahme der beim Einlegen eines Schlauches (80) verdrängten, von der Membran (30) eingeschlossenen Flüssigkeit (50) vorgesehen sind.

l0. Ultraschallsensor nach Anspruch 9, dadurch gekennzeichnet, daß die Nuten (l6) in den Seitenwänden (l2, l3) rechteckigen Querschnitt besitzen.

ll. Ultraschallsensor nach Anspruch 3 sowie einem der Ansprüche 9 oder l0, dadurch gekennzeichnet, daß die Nuten (l7) im Gehäuseboden (ll) dreieckigen Querschnitt besitzen.

l2. Ultraschallsensor nach einem der Ansprüche 2 bis ll, dadurch gekennzeichnet, daß bei beiden Gehäusehälften (l0) oberhalb der Membran (30) Führungsleisten (l8) vorgesehen sind, welche von den Seitenwänden (l2, l3) nach innen weisen.

l3. Ultraschallsensor nach Anspruch l2, dadurch gekennzeichnet, daß die Führungsleisten (l8) bis an den eingelegten Schlauch (80) seitlich heranreichen.

l4. Ultraschallsensor nach Anspruch l2 oder l3, dadurch gekennzeichnet, daß die Führungsleisten - (l8) in Längsrichtung gebogen sind.

l5. Ultraschallsensor nach einem der Ansprüche l bis l4, dadurch gekennzeichnet, daß die Membran (30) aus einem gummielastischen Kunststoff besteht.

l6. Ultraschallsensor nach einem der Ansprüche l bis l5, dadurch gekennzeichnet, daß die beiden Gehäusehälften (l0) mittels eines Gelenks - (60) aufklappbar miteinander verbunden sind.

l7. Ultraschallsensor nach Anspruch l6, dadurch gekennzeichnet, daß an den dem Gelenk (60) gegenüberliegenden Seiten der Gehäusehälften (l0) ein Verschluß (70) angebracht ist.

# Fig.1

# Fig. 2

# Fig.3

# Fig.4

# Fig.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-2 732 550 (RENAL SYSTEM, INC.) * ganze Schrift * | 1,2 | G 01 N 29/02 G 10 K 11/00 A 61 M 5/14 |
| | --- | | |
| Y | DE-A-3 503 477 (K.K. TOSHIBA) * Seite 8, Zeilen 5-28; Seite 11, Zeilen 4-14; Figur 4 * | 1,2 | |
| | --- | | |
| A | GB-A-1 418 181 (E.M. COLE) * ganze Schrift * | 1 | |
| | --- | | |
| A | US-A-3 974 681 (J. NAMERY) * ganze Schrift * | 1 | |
| | --- | | |
| A,D | EP-A-0 053 453 (BAXTER TRAVENOL LABORATORIES, INC.) * ganze Schrift * | 1 | |
| | ----- | | |

**RECHERCHIERTE SACHGEBIETE (Int. Cl.4)**

G 01 N 29/02
G 10 K 11/00
A 61 M 5/14

Der vorliegende Recherchenbericht wurde für alle Patentansprüche erstellt.

| Recherchenort | Abschlußdatum der Recherche | Prüfer |
|---|---|---|
| BERLIN | 28-11-1986 | BRISON O.F. |